# FASCICULE DE BREVET EUROPEEN

(11) **EP 0 674 901 B1**
(45) Date de publication et mention de la délivrance du brevet: **25.11.1998**
(21) Numéro de dépôt: 95400574.0
(22) Date de dépôt: 15.03.1995
(51) Int. Cl.: A61K 9/70

(54) **Système matriciel transdermique d'administration d'un oestrogène et/ou un progestatif à base de copolymère styrène-isoprène-styrène**
Transdermales Matrixsystem auf Basis eines Styrol-Isopren-Styrol Copolymers zur Verabreichung eines Östrogens und/oder Gestagens
Transdermal matrix system comprising a styrene-isoprene-styrene copolymer for the administration of estrogen and/or progestin

(30) Priorité: 28.03.1994 FR 9403599
(43) Date de publication de la demande: 04.10.1995
(73) Titulaire: LABORATOIRES D'HYGIENE ET DE DIETETIQUE, 75008 Paris (FR)
(72) Inventeur: Mikler, Claude, F-21000 Dijon (FR); Liorzou, Laurent, 23, chemin de Nicol,F-31200 Toulouse (FR); Dhuique-Mayer, Daniel, F-21000 Dijon (FR)
(74) Mandataire: Clisci, Serge

(56) Documents cités:
- EP-A- 0 156 565
- EP-A- 0 483 370
- DATABASE WPI Week 9403, Derwent Publications Ltd., London, GB; AN 94-022826 & JP-A-5 331 064 (SEKISUI CHEM IND CO LTD) 14 Décembre 1993

## Description

### Domaine de l'invention

La présente invention a pour objet un nouveau dispositif ou système matriciel transdermique pour la libération prolongée d'un composant oestrogène et/ou d'un composant progestatif, ledit dispositif étant formé d'un support et d'une matrice auto-adhésive, qui est composée d'un copolymère tribloc présentant des unités A-B-A du type poly(styrène-isoprène-styrène) [en abrégé : SIS], et dans laquelle sont dissous ledit composant oestrogène et/ou ledit composant progestatif, en association avec un couple plastifiants/promoteurs. L'invention concerne également un procédé de préparation dudit système matriciel.

### Art antérieur

On sait que nombreux systèmes transdermiques devant libérer un composant oestrogène et/ou un composant progestatif ont déjà été proposés. Parmi ceux-ci on trouve aujourd'hui des systèmes dits "réservoirs" dans lesquels le principe actif est dissous dans un solvant servant de vecteur de transport à travers une membrane microporeuse vers la peau. Tel est le cas des dispositifs à base (i) de 17-[3-oestradiol et (ii) de 17-[3-oestradiol associé à de l'acétate de noréthistérone, commercialisés par la société CIBA-GEIGY respectivement sous les dénominations ESTRADERM^{@} TTS et ESTRAGEST^{@} TTS.

Parallèlement il existe des systèmes dits matriciels dans lesquels les principes actifs sont dissous ou dispersés au sein d'une matrice adhésive à base de polymères tels des copolymères EVA, acryliques, poly(styrène-isoprène-styrène) etc.

Tous ces systèmes, si l'on désire obtenir un produit final thérapeutiquement efficace, doivent impérativement posséder un niveau d'administration de principes actifs pendant une période prolongée à un débit suffisant pour l'obtention de taux plasmatiques adaptés aux besoins thérapeutiques. De plus pour être bien acceptés par le patient, ils doivent présenter des dimensions relativement réduites et des propriétés d'adhésion et de cohésion optimales, afin de permettre un usage commode sans altération des vêtements par fluage de la masse de la matrice pendant l'utilisation.

Or, il est connu de l'homme de l'art que les composants oestrogènes et les composants progestatifs sont des produits (i) qui sont peu solubles dans les polymères utilisés dans les systèmes transdermiques, et (ii) qui passent difficilement la barrière cutanée.

Aussi les quantités libérées de ces principes actifs pour obtenir l'effet thérapeutique recherché sont en général faibles par rapport aux quantités initiales présentes dans les dispositifs transdermiques ce qui a pour conséquence l'obtention de rendements faibles. Ceci entraîne la mise en oeuvre d'une quantité d'hormone(s) très excédentaire par rapport à celle effectivement consommée.

Dans ce cas un compromis entre un taux d'administration transdermique efficace d'une ou plusieurs hormones, d'une part, et de bonnes propriétés physiques et ergonomiques des systèmes, d'autre part, est difficile à atteindre.

Bien que de nombreuses formulations à base de copolymère tribloc SIS soient connues de EP-A-0 439 180, EP-A-0 285 181 ou EP-A-0 483 370, aucune de ces publications ne divulgue ni ne suggère les formulations spécifiques de l'invention.

### Buts de l'invention

Dans le domaine de l'administration par voie transdermique d'un composant oestrogène et/ou d'un composant progestatif, il serait souhaitable de disposer d'une nouvelle solution technique faisant appel à un système matriciel dans lequel la matrice contient un copolymère tribloc SIS, et permettant de réaliser le compromis recherché sans les inconvénients précités avec en plus d'excellents rendements.

Selon un premier aspect de l'invention, on se propose de fournir un système matriciel transdermique, dans lequel la matrice comporte un matériau SIS, pour l'administration d'un composant oestrogène et/ou d'un composant progestatif.

Selon un second aspect on se propose de fournir un procédé pour la préparation d'un tel système.

### Objet de l'invention

Les buts précités sont obtenus grâce à une nouvelle solution technique selon laquelle la matrice du système matriciel, qui contient un composant oestrogène et/ou un composant progestatif, est essentiellement constituée d'un matériau SIS et d'un couple plastifiant/promoteur.

Selon l'invention, on préconise en premier lieu, en tant que produit industriel nouveau, un système matriciel transdermique pour l'administration percutanée d'une hormone, ledit système matriciel, qui comporte un support et une matrice auto-adhésive, étant caractérisé en ce que ladite matrice comprend :
(a) 20 à 42 parties en poids d'un copolymère tribloc poly(styrène-isoprène-styrène),
(b) 35 à 55 parties en poids d'une résine tackifiante,
(c) 5 à 25 parties en poids d'un agent plastifiant choisi parmi l'alcool oléique, le 5-oléate de péglicol, le laurate de propylèneglycol ou un éther polypropoxylé de l'alcool cétylique,
(d) 5 à 15 parties en poids d'au moins un composé choisi parmi
   - le crotamiton, et
   - les 2-pyrrolidones N-substituées de formule I, 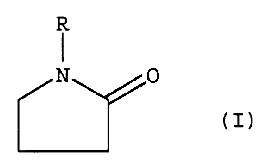 où R représente un groupe alkyle en C₁-C₁₅, le groupe cyclohexyle ou le groupe 2-hydroxyéthyle,
(e) 0,01 à 1 partie en poids d'un agent stabilisant, et,
(f) 0,1 à 5 parties en poids d'une hormone choisie parmi l'ensemble constitué par les composants oestrogènes, les composants progestatifs et leurs mélanges.

En second lieu on préconise un procédé pour la préparation dudit système matriciel transdermique, ledit procédé étant caractérisé en ce qu'il comprend les étapes suivantes qui consistent soit, à
(a) mélanger le polymère SIS, l'agent stabilisant et la résine tackifiante à une température supérieure à 110°C, puis homogénéiser le mélange résultant;
(β) incorporer dans le mélange homogène ainsi obtenu le ou les promoteurs et le plastifiant à une température comprise entre 80 et 110°C, puis homogénéiser le mélange résultant;
(y) incorporer dans le mélange homogène ainsi obtenu l'hormone choisie parmi l'ensemble constitué par les composants oestrogènes, les composants progestatifs et leurs mélanges, puis homogénéiser le mélange résultant;
(&) enduire le mélange homogène ainsi obtenu, à une température comprise entre 80 et 130°C, sur un support temporaire antiadhérent de façon à obtenir un revêtement sur ledit support de 50 à 300 g/m²; et,
(e) transférer ledit revêtement sur un support définitif;
   soit à,
   (a) mélanger le polymère SIS, l'agent stabilisant puis la résine tackifiante dans un solvant dudit polymère SIS, à une température inférieure à la température d'ébullition dudit solvant, puis homogénéiser le mélange résultant;
   (β) incorporer dans le mélange ainsi obtenu le ou les promoteurs et le plastifiant, puis homogénéiser le mélange résultant;
   (y) incorporer dans le mélange homogène ainsi obtenu l'hormone choisie parmi l'ensemble constitué par les composants oestrogènes, les composants progestatifs et leurs mélanges, puis homogénéiser le mélange résultant;
   (&) enduire le mélange homogène ainsi obtenu, à température ambiante, sur un support temporaire antiadhérent de façon à obtenir un revêtement sur ledit support de 50 à 300 g/m²; et,
   (s) évaporer le solvant en chauffant ledit revêtement à une température supérieure au point d'ébullition dudit solvant, puis transférer ledit revêtement sur un support définitif.

### Dessins

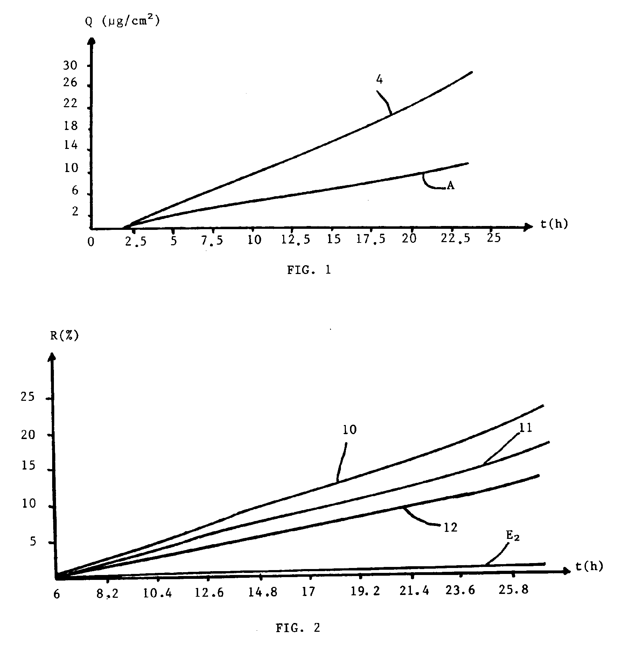

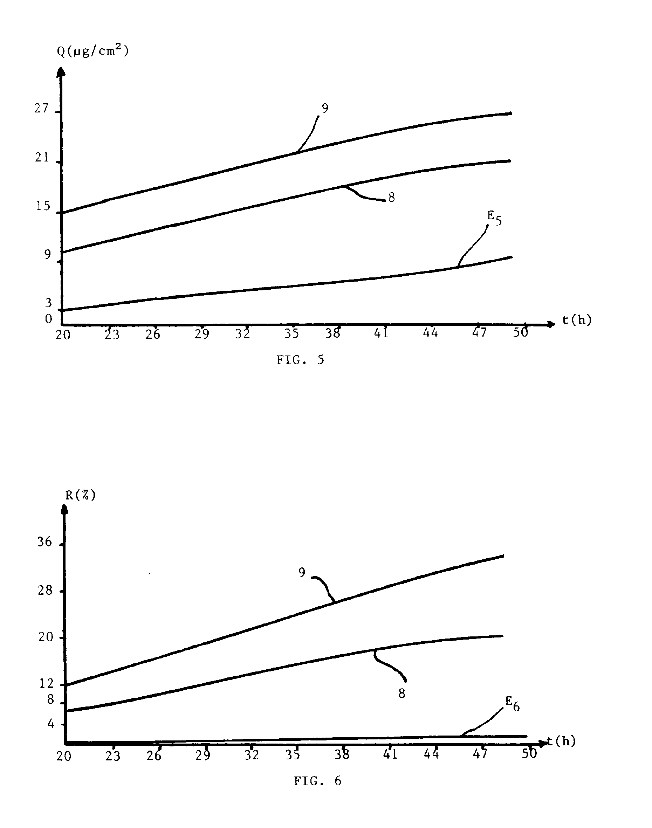

Dans les dessins annexés, les figures 1 et 5 représentent la quantité (Q), exprimée en wg/cm², de 17-p-oestradiol libérée en fonction du temps (t), exprimé en heures; et les figures 2-4 et 6 représentent le rendement (R), exprimé en %, en 17-β-oestradiol ou NETA (acétate de noréthistérone) en fonction du temps (t), exprimé en heures.

Plus précisément dans ces dessins,
- la figure 1 permet de comparer (dans le système Q/t) les courbes 4 et A relatives à la libération de 17-β-oestradiol et obtenues respectivement avec le produit de l'exemple 4 selon l'invention et le produit de comparaison CP4;
- la figure 2 permet de comparer (dans le système R/t) les courbes 10, 11, 12 et E₂ relatives au rendement de la libération de 17-p-oestradiol et obtenues respectivement avec les produits des exemples 10, 11 et 12 selon l'invention et un produit transdermique connu sous la dénomination ESTRAGEST^{®} et commercialisé par la société CIBA-GEIGY;
- la figure 3 permet de comparer (dans le système R/t) les courbes 10, 11, 12 et E₃ relatives au rendement de la libération de NETA et obtenues respectivement avec les produits des exemples 10, 11 et 12 selon l'invention et ledit ESTRAGEST^{®};
- la figure 4 permet de comparer (dans le système R/t) les courbes 5 et E4 relatives au rendement de la libération de 17-p-oestradiol et obtenues respectivement avec le produit de l'exemple 5 selon l'invention et ledit ESTRA-GEST^{@};
- la figure 5 permet de comparer (dans le système Q/t) les courbes 8, 9 et E5 relatives à la libération de 17-p-oestradiol et obtenues respectivement avec les produits des exemples 8 et 9 selon l'invention et ledit ESTRAGEST^{@};
- la figure 6 permet de comparer (dans le système R/t) les courbes 8, 9, et E₆ relatives au rendement de la libération de NETA et obtenues respectivement avec les produits des exemples 8 et 9 selon l'invention et ledit ESTRA-GEST^{@}.

### Description détaillée de l'invention

Par copolymère tribloc poly(A-B-A) du type poly(styrène-isoprène-styrène), on entend ici un matériau SIS ayant une teneur en styrène comprise entre 14 et 50 % en poids par rapport au poids dudit SIS. On utilisera de préférence un SIS contenant 17 à 47 % en poids de styrène. Il est également possible d'utiliser des mélanges de ces SIS.

Parmi les résines tackifiantes (ou poisseuses) qui conviennent selon l'invention, on peut mentionner les résines généralement employées dans le domaine des adhésifs par l'homme de l'art telles les résines polyterpènes ou terpènes modifiées, les résines de colophane hydrogénée, de colophane polymérisée, les résines d'esters de colophane, etc.

On préférera en particuliers les résines hydrocarbonées telles que celle commercialisée par la société EXXON CHEMICAL sous la dénomination ESCOREZ^{@-}385. Ces résines permettent en effet, en association avec les SIS préférés selon l'invention, d'obtenir des propriétés adhésives optimales.

Par principe "actif" ou "hormone" on entend ici tout composant oestrogène, tout composant progestatif ou tout mélange composant oestrogène/composant progestatif utilisable par voie transdermique.

Parmi les composants oestrogènes qui conviennent selon l'invention on peut citer en particulier le 17-β-oestradiol, et les dérivés de l'oestradiol, notamment les mono- et di-esters de l'oestradiol, comme par exemple le 17-acétate oestradiol, le 3,17-diacétate oestradiol, le 3-benzoate oestradiol, le 17-undécanoate oestradiol, les dérivés alkylés en position 17 de l'oestradiol tel que l'éthinyloestradiol, le 3-isopropylsulfonate éthinyloestradiol, le méthyloestradiol, le quinestrol, le mestranol et, le cas échéant, leurs mélanges.

Parmi les composants progestatifs qui conviennent selon l'invention on peut citer en particulier la progestérone, la médrogestérone et leurs dérivés (notamment l'acétate de 17-hydroxyprogestérone, l'acétate de médroxyprogesté- rone), la noréthistérone et ses dérivés notamment l'acétate de 17-noréthistérone.

Selon la présente invention, on utilisera de préférence comme composant oestrogène le 17-β-oestradiol et comme composant progestatif l'acétate de noréthistérone (NETA). Selon une variante de réalisation, le système matriciel transdermique selon l'invention peut contenir simultanément un composant oestrogène et un composant progestatif.

Parmi les agents stabilisants, utilisés selon l'invention, on peut citer les agents antioxydants couramment employés par l'homme de l'art comme par exemple les produits commercialisés par la société CIBA-GEIGY sous la dénomination IRGANOX^{®} 565 ou IRGANOX^{®} B215.

Le promoteur, moyen (d), peut être choisi parmi le crotamiton, les composés N-alkyl-2-pyrrolidones, où le groupe alkyle est en C₁-C₁₅, la N-(2-hydroxyéthyl)-2-pyrrolidone, la N-cyclohexyl-2-pyrrolidone et leurs mélanges.

Parmi les N-alkyl-2-pyrrolidones utilisés dans la présente invention, où le groupe alkyle est en C₁-C₁₅ on préférera la N-méthyl-2-pyrrolidone, la N-éthyl-2-pyrrolidone, la N-octyl-2-pyrrolidone, la N-dodécyl-2-pyrrolidone, commercialisées par la société GAF CORPORATION respectivement sous les dénominations NMP^{@}, NEP^{@}, SURFADONE^{@} LP 100 et SURFADONE^{®} LP 300, et leurs mélanges.

Quand le plastifiant est un éther polypropoxylé de l'alcool cétylique on préférera un composé contenant notamment 10 moles d'oxyde de propylène pour une mole d'alcool cétylique comme par exemple le produit commercialisé par la société CRODA FRANCE S.A. sous la dénomination PROCETYL^{®}10.

Le support recevant la matrice pourra être tout support généralement utilisé dans les systèmes transdermiques occlusifs ou non, d'épaisseur variable, qui est imperméable aux constituants de la matrice. On préférera par exemple un support sous forme de film en polyéthylène, polypropylène, polyester, un complexe (ou composite) constitué de polyéthylène et d'un copolymère d'acétate de vinyle et d'éthylène, ou encore des mousses.

De façon pratique, la surface de la matrice, qui n'est pas liée au support, pourra être recouverte d'une couche ou pellicule de protection pelable avant utilisation du dispositif; ledit dispositif pourra être lui-même emballé dans une protection étanche comme par exemple les complexes polyéthylène-aluminium.

Grâce à la spécificité de la composition des formulations pouvant former la matrice, seul le système matriciel selon l'invention présente les nombreux avantages que l'on va maintenant exposer.

Il a été en effet trouvé que seule la composition définie ci-dessus contenant (1) un couple plastifiant/promoteur, où (i) l'agent plastifiant, représente l'alcool oléique, le 5-oléate de péglicol, le laurate de propylèneglycol ou un éther polypropoxylé de l'alcool cétylique, et (ii) le promoteur est au moins un composé choisi parmi les 2-pyrrolidones N-substituées de formule et le crotamiton, et (2) un matériau SIS, permet d'obtenir à la fois des rendements remarquables et les propriétés adhésives et cohésives souhaitées.

En effet, on n'obtient pas de résultats identiques avec une matrice à base d'un autre type de copolymère comme par exemple un copolymère d'éthylène et d'acétate de vinyle (EVA) associé à ce couple plastifiant/promoteur.

Ceci s'explique sans doute par (i) un effet synergique particulier entre la nature des copolymères triblocs poly(styrène-isoprène-styrène) qui ont tendance à "repousser" la ou les hormones présentes, qui sont insolubles dans ces derniers, et (ii) le rôle des agents plastifiants spécifiques qui vont écarter les chaînes polymériques de SIS, leur permettant de plus grands mouvements, et donc de diminuer la rigidité du réseau macromoléculaire ce qui facilite au total la circulation de la ou des hormones.

Mais ces plastifiants qui sont des dérivés de corps gras peuvent, utilisés en trop grande quantité, altérer les propriétés adhésives et/ou cohésives de la matrice. Aussi, il est essentiel qu'ils soient associés avec un ou des promoteurs de la perméation cutanée de nature différente telle crotamiton ou les composés 2-pyrrolidones N-substituées de formule I pour obtenir les taux d'administration recherchés et parvenir à un meilleur rendement sans perte d'adhésion ou de cohésion.

Lesdits crotamiton et 2-pyrrolidones N-substituées de formule 1 permettent aussi d'améliorer la solubilité des hormones, que l'on utilise, dans la matrice.

Ces formulations spécifiques conduisent à des systèmes transdermiques auto-adhésifs, possédant une bonne cohésion, une bonne adhésion et présentant des rendements à 24 heures ou 48 heures en composant oestrogène de l'ordre de 10 à 84 % et en composant progestatif de l'ordre de 3,5 à 32 %.

La possibilité d'utiliser moins de composant oestrogène et/ou de composant progestatif tout en obtenant des quantités administrées plus importantes simplifie la mise au point et la réalisation des formulations formant la matrice des dispositifs.

On minimise ou supprime ainsi les problèmes de solubilité des hormones dans les copolymères SIS ainsi que les risques d'incompatibilité physique avec les autres constituants de la matrice. Il en est de même des problèmes de cristallisation des hormones et de l'instabilité des dispositifs au cours du temps, ces phénomènes étant inacceptables pour la validation et la commercialisation de produits à visée thérapeutique tels des systèmes transdermiques.

Un dernier avantage non négligeable est celui du prix de revient, très nettement abaissé par rapport aux dispositifs connus de l'art antérieur, grâce à l'utilisation d'une faible quantité d'hormone dont le prix est élevé.

Les systèmes matriciels transdermiques selon l'invention sont réalisés suivant les techniques généralement employées par l'homme de l'art : soit par enduction (en phase solvant), soit selon la technique dite "hot-melt" (c'est-à-dire en l'absence de solvant).

Dans les deux cas, dans le cadre d'une production industrielle, on enduit de grandes surfaces qui sont ensuite découpées pour donner des dispositifs aux dimensions adaptées, en fonction de la quantité d'hormone(s) présente par unité de surface, à la dose choisie de principe actif à administrer pendant un temps déterminé.

Dans le cas de la technique dite en phase solvant, on préconise un procédé pour la préparation d'un système matriciel transdermique auto-adhésif selon l'invention qui comprend les étapes suivantes :
(1) dans un réacteur thermostaté contenant un solvant du polymère, on introduit en chauffant à une température inférieure au point d'ébullition du solvant (par exemple 60°C dans le cas de l'acétate d'éthyle) le copolymère SIS, le stabilisant puis la résine tackifiante et on agite jusqu'à complète homogénéisation du mélangé;
(2) on incorpore toujours sous agitation en chauffant à la même température tous les composés liquides c'est-à-dire le ou les promoteurs et le plastifiant, et on homogénéise;
(3) le ou les principes actifs, soit directement en poudre, soit en solution dans un solvant du composant oestrogène et/ou du composant progestatif comme par exemple le tétrahydrofuranne, sont incorporés au mélange obtenu à l'étape (2), toujours sous agitation, en chauffant à la même température jusqu'à complète homogénéisation;
(4) on enduit le mélange homogène ainsi obtenu à l'étape (3), à température ambiante, sur un support temporaire non-adhérent, par exemple une pellicule de polyester siliconé, à raison de 50 à 300 g/m²;
(5) l'enduction est chauffée pour évaporer le solvant à une température, fonction du point d'ébullition de ce dernier, comprise entre 40 et 110°C, et de préférence une température de 60 à 80°C; et,
(6) on transfère la matrice sèche résultante sur le support final choisi.

Dans le cas de la technique "hot-melt" on préconise un procédé qui comprend les étapes suivantes :
(1a) dans un malaxeur, on incorpore à l'ensemble polymère SIS/stabilisant, sous agitation, à une température supérieure à 110°C, de préférence une température de 130°C, la résine tackifiante par portions successives de 10 %, 30 % et 60 %, de manière qu'à chaque portion on aboutisse à un mélange parfaitement homogène;
(2a) on incorpore ensuite progressivement au mélange obtenu à l'étape (1 a) les produits plus liquides de la formulation c'est-à-dire le plastifiant et le ou les promoteurs toujours sous agitation et à des températures généralement plus faibles qu'à l'étape (1a) déterminées par la stabilité thermique de ces produits; on agite à nouveau jusqu'à complète homogénéisation du mélange résultant;
(3a) on incorpore le ou les principes actifs dans le mélange homogène ainsi obtenu à l'étape (2a), à une température de l'ordre de 100°C, et on agite encore jusqu'à obtenir un mélange parfaitement homogène;
(4a) on enduit le mélange homogène ainsi obtenu, à une température comprise entre 80 et 130°C, sur un support temporaire non-adhérent, notamment une pellicule de polyester siliconé, pour obtenir un dépôt de 50 à 300 g/m²; et,
(5a) on transfère la matrice ainsi obtenue à l'étape (4a) sur le support final choisi.

Les systèmes transdermiques selon l'invention sont en particulier utiles pour le traitement des symptômes de la ménopause et des risques cardio-vasculaires qui en découlent, l'ostéoporose ainsi que pour toute thérapie utilisant la voie percutanée nécessitant l'administration d'oestrogènes et/ou de progestatifs.

### Meilleur mode

Le meilleur mode de mise en oeuvre de l'invention consiste à faire appel à un système matriciel transdermique dont la matrice contient, pour un total de 100 parties en poids :
(a) 37,3 parties en poids de copolymère tribloc poly(styrène-isoprène-styrène),
(b) 38 parties en poids de résine tackifiante,
(c) 15 parties en poids de laurate de propylène glycol,
(d) 7 parties en poids de N-octyl-2-pyrrolidone,
(e) 0,2 partie en poids d'agent stabilisant,
(f) 0,5 partie en poids de 17-p-oestradiol,
(g) 2 parties en poids d'acétate de noréthistérone,

D'autres avantages et caractéristiques de l'invention seront mieux compris à la lecture de la description qui va suivre d'exemples de réalisation et d'essais comparatifs.

Bien entendu l'ensemble de ces éléments n'est nullement limitatif mais est donné à titre d'illustration.

Par commodité, dans ce qui suit les abréviations ont été utilisées :
NETA : acétate de noréthistérone
SIS : copolymère tribloc poly(styrène-isoprène-styrène)
EVA : copolymère d'éthylène et d'acétate de vinyle.

### Exemple 1:

Dans un bécher de 250 ml, on introduit 13,9 g de VECTOR^{@} 4211 D (copolymère SIS commercialisé par la société EXXON CHEMICAL), 23 g de ECR^{@} 385 (résine tackifiante commercialisée par la société EXXON CHEMICAL), 9 g de PROCETYL^{@} 10 (éther polypropoxylé de l'alcool cétylique commercialisé par la société CRODA FRANCE SA), 3 g de SURFADONE^{@} LP 100 (N-octyl-2-pyrrolidone, commercialisée par la société GAF CORPORATION), 0,1 g d'IRGA-NOX^{@} 565 (antioxydant commercialisé par la société CIBA-GEIGY) et 19,65 g d'acétate d'éthyle. On agite ce mélange en chauffant à 60°C jusqu'à dissolution complète des composés. On ajoute alors une solution de 1 g de NETA préalablement dissous dans 5 g de tétrahydrofuranne. On agite le mélange résultant ainsi obtenu, durant environ 30 minutes, jusqu'à complète homogénéisation. On laisse refroidir et dégazer au repos jusqu'à la disparition totale des bulles. On enduit la masse résultante sur une pellicule de polyester siliconé à raison de (110 ± 10) g/m², à température ambiante (15-20°C). L'enduction ainsi réalisée est chauffée à 50°C pendant au moins 30 minutes puis transférée sur un support polyéthylène non siliconé. On découpe alors des formes de dimensions appropriées que l'on conditionne dans des sachets.

On obtient un système dont la quantité initiale en NETA est de 231 µg/cm².

### Exemple 2

On procède de façon identique à l'exemple 1, mais en utilisant ici 13,4 g de VECTOR^{®} 4211 D, 0,1 g d'IRGANOX^{®} 565, 26,5 g de ECR^{®} 385, 5 g d'alcool oléique, 4,5 g de crotamiton [N-éthyl-N(2-méthylphényl)-2-butenamide, commercialisé par la société BOEHRINGER INGELHEIM], 20 g d'acétate d'éthyle et 0,5 g de 17-β-oestradiol dissous dans 2,5 g de tétrahydrofuranne. On enduit à raison de (100 ± 10) g/m².

On obtient un système dont la quantité initiale en 17-p-oestradiol est de 94,5 µg/cm².

### Exemple 3

On procède de façon identique à l'exemple 1, mais en utilisant ici 13,9 g de VECTOR® 4211 D, 0,1 g d'IRGANOX® 565, 27,5 g de ECR⁸ 385, 5 g d'alcool oléique, 3 g de SURFADONE® LP 300, 20,8 g d'acétate d'éthyle et 0,5 g de 17-β-oestradiol dissous dans 2,5 g de tétrahydrofuranne. On enduit à raison de (100 ± 10) g/m².

On obtient un système dont la quantité initiale en 17-p-oestradiol est de 91 µg/cm².

### Exempte 4

On procède de façon identique à l'exemple 1, mais en utilisant 7,25 g de VECTOR® 4211 D, 0,07 g d'IRGANOX® 565, 11g de ECR® 385, 3,75 g de LABRAFIL® 1944Cs (5-oléate de péglicol commercialisé par la société GATTEFOSSE), 2,5 g de crotamiton, 10 g d'acétate d'éthyle et 0,5 g de 17-β-oestradiol dissous dans 2,5 g de tétrahydrofuranne. On enduit à raison de (90 ± 10) g/m².

On obtient un système dont la quantité initiale en 17-p-oestradiol est de 181,7 µg/cm².

### Exempte 5

On procède de façon analogue à l'exemple 4, mais en utilisant 12,9 g de VECTOR® 4211 D, 0,1 g d'IRGANOX® 565, 26 g de ECR® 385, 8 g de LABRAFIL® 1944Cs, 2,5 g de SURFADONE® LP 300, 19,4 g d'acétate d'éthyle et 0,5 g de 17-p-oestradiol dissous dans 2,5 g de tétrahydrofuranne. On enduit à raison de (100 ± 10) g/m².

On obtient un système dont la quantité initiale de 17-β-oestradiol est de 87,1 µg/cm².

### Exempte 6

On procède de façon analogue à l'exemple 2, mais en utilisant 13,16 g de VECTOR^{®} 4211 D, 0,1 g d'IRGANOX® 565, 26,5 g de ECR ^{®} 385, 5,08 g d'alcool oléique, 4,53 g de crotamiton, 19,8 g d'acétate d'éthyle et 0,75 g de 17-β-oestradiol dissous dans 3,75 g de tétrahydrofuranne. On enduit à raison de (100 ± 10) g/m².

On obtient un système dont la quantité initiale de 17-p-oestradiol est de 159 µg/cm².

### Exemple 7

On procède de façon analogue à l'exemple 3 en utilisant 0,75 g de 17-p-oestradiol dissout dans 3,75 g de tétrahydrofuranne, 13,64 g de VECTOR^{®} 4211D, 0,1 g d'IRGANOX®565, 27,51 g de ECR^{®} 385, 5 g d'alcool oléique, 3 g de SURFADONE^{®} LP 300 et 20,5 g d'acétate d'éthyle. On enduit à raison de (100 ± 10) g/m².

On obtient un système dont la quantité initiale de 17-β-oestradiol est de 162,3 µg/cm².

### Exemple 8

On procède de façon analogue à l'exemple 1, mais en utilisant 13,9 g de VECTOR^{®} 4211 D, 0,1 g d'IRGANOX® 565, 24 g de ECR^{®} 385, 3,5 g de SURFADONE^{®} LP 300, 7,5 g de LAUROGLYCOL® (mélange de mono et di-ester de propylèneglycol et d'acide laurique commercialisé par la société GATTEFOSSE) à la place du PROCETYL^{®} 10, 19,9 g d'acétate d'éthyle, 0,25 g de 17-β-oestradiol et 0,75 g de NETA dissous dans 5 g de tétrahydrofuranne. On enduit à raison de (100 ± 10) g/m².

On obtient un système dont la quantité initiale de 17-p-oestradiol est de 48,6 µg/cm² et la quantité initiale de NETA est de 145,3 µg/cm².

### Exemple 9

On procède de façon identique à l'exemple 8, mais en utilisant 15,9 g de VECTOR^{®} 4211D, 0,1 g d'IRGANOX^{®} 565, 21 g de ECR^{®} 385, 7,5 g de LAUROGLYCOL^{®}, 4,5 g de crotamiton (en remplacement de la SURFADONE^{®} LP 300), 21,15 g d'acétate d'éthyle, 0,25 g de 17-β-oestradiol et 0,75 g de NETA dissous dans 5 g de tétrahydrofuranne. On enduit à raison de (70 ± 10) g/m².

On obtient un système dont la quantité initiale de 17-β-oestradiol est de 33,1 µg/cm² et la quantité initiale de NETA est de 99,4 µg/cm².

### Exemple 10

On procède de façon identique à l'exemple 1, mais en utilisant ici 14,9 g de VECTOR^{®} 4211D, 0,11 g d'IRGA-NOX^{©} 565, 23,25 g de ECR^{©} 385, 7 g de PROCETYL^{©} 10, 3,56 g de SURFADONE^{©} LP 100, 20,7 g d'acétate d'éthyle, 0,25 g de 17-β-oestradiol et 1 g de NETA dissous dans 6,25 g de tétrahydrofuranne. On enduit à raison de (100 ± 10) g_{/m}2_{.}

On obtient un système dont la quantité initiale de 17-p-oestradiol est de 51 µg/cm² et la quantité initiale de NETA est de 207,1 µg/cm².

### Exemple 11

On procède de façon identique à l'exemple 10, mais on remplace la SURFADONE^{®} LP 100 par 3,5 g de SURFA-DONE^{®} LP 300. On enduit à raison de (120 ± 10) g/m².

On obtient un système dont la quantité initiale de 17-p-oestradiol est de 60,5 µg/cm² et la quantité initiale de NETA est de 242 µg/cm².

### Exemple 12

On procède de façon identique à l'exemple 10, mais en utilisant 14,65 g de VECTOR^{®} 4211 D, 0,1 g d'IRGANOX^{®} 565, 21,75 g de ECR^{®} 385, 7 g de PROCETYL® 10, 5 g de crotamiton, 20,1 g d'acétate d'éthyle, 0,25 g de 17-p-oestradiol et 1 g de NETA dissous dans 6,25 g de tétrahydrofuranne. On enduit à raison de (120 ± 10) g/m².

On obtient un système dont la quantité initiale de 17-β-oestradiol est de 60,5 µg/cm2 et la quantité initiale de NETA est de 235,4 µg/cm².

### Exemple 13

On procède de façon analogue à l'exemple 8 mais en utilisant 18,65 g de VECTOR^{@} 4211D, 0,1 g d'IRGANOX^{®} 565, 19 g de ECR^{®} 385, 3,5 g de SURFADONE^{®} LP 100, 7,5 g de LAUROGLYCOL^{®}, 23 g d'acétate d'éthyle et dissous dans 5 g de tétrahydrofuranne, 0,25 g de 17-β-oestradiol et 0,75 g de NETA. On enduit à raison de (100 ± 10) g_{/m}².

On obtient un système dont la quantité initiale de 17-p-oestradiol est de 47,3 µg/cm² et la quantité initiale de NETA est de 190 µg/cm².

On a réalisé dans un but comparatif les matrices suivantes à base d'EVA.

### PRODUIT DE COMPARAISON CP1

Dans un bécher de 250 ml, on introduit 29 g de LEVAPREN^{®} 450P (EVA commercialisé par la société BAYER), 15,5 g de PROCETYL® 10, 3,5 g de SURFADONE^{®} LP 100 et 67,7 g d'acétate d'éthyle. On chauffe à 60°C en agitant pendant 5 heures jusqu'à dissolution complète de l'EVA.

On ajoute alors 2 g de NETA préalablement dissous dans 10 g de tétrahydrofuranne. On agite le mélange ainsi obtenu, en chauffant durant environ 30 minutes jusqu'à complète homogénéisation. On laisse le mélange ainsi obtenu refroidir et dégazer au repos jusqu'à disparition totale des bulles. On enduit à la température ambiante la masse résultante sur papier siliconé à raison de (120 ± 10) g/m². L'enduction ainsi réalisée est chauffée à 70°C pendant au moins 30 minutes puis transférée sur un support définitif en polyéthylène non siliconé. On découpe alors des formes de dimensions appropriées que l'on conditionne en sachets.

On obtient un système dont la quantité initiale de NETA est de 488 ¿g/cm².

### PRODUIT DE COMPARAISON CP2

On procède de façon analogue à CP1, mais en utilisant ici 29 g de LEVAPREN^{@} 450P, 16,5 d'alcool oléique, 3,5 g de crotamiton, 67,6 g d'acétate d'éthyle et 1 g de 17-p-oestradiol dissous dans 5 g de tétrahydrofuranne. On enduit à raison de (80 ± 10) g/m².

On obtient un système dont la quantité initiale de 17-0-oestradiol est de 157 µg/cm².

### PRODUIT DE COMPARAISON CP3

On procède de façon analogue à CP2, mais en utilisant 1 g de 17-β-oestradiol dissous dans 5 g de tétrahydrofuranne, 29 g de LEVAPREN^{@} 450P, 16,25 g d'alcool oléique, 67,6 g d'acétate d'éthyle et 3,75 g de SURFADONE^{O} LP 300. On enduit à raison de (80 ± 10) g/m².

On obtient un système dont la quantité initiale de 17-p-oestradiol est de 169,6 µg/cm².

### PRODUIT DE COMPARAISON CP4

On utilise ici une formulation à base d'EVA du même type que celles décrites dans la demande publiée FR-A-2 612 785 précitée.

On introduit dans un bécher 21,97 g de LEVAPREN^{@} 450P, 12,54 g d'EUTANOL^{®} G (2-octyl-dodécanol, commercialisé par la société HENKEL). On chauffe à 130°C et on agite ce mélange auquel on additionne 6,03 g d'ETHOCEL^{@} 20 (éthylcellulose commercialisée par la société DOW CHEMICAL) puis 6,46 g d'EUTANOL^{®} G. On mélange ainsi jusqu'à obtention d'un mélange homogène et sans grains et on laisse refroidir. On porte ensuite la masse à 80°C et on additionne une solution de 1,5 g de dipropylène glycol et 1,5 g de 17-p-oestradiol préalablement dissous à 70°C dans 12 g d'éthanol anhydre. On mélange l'ensemble pendant au moins une heure jusqu'à complète homogénéisation. On enduit cette masse sur un support papier siliconé à une température de 70°C à raison de (150 ± 10) g/m². On sèche à 80°C pour évaporer l'éthanol puis on transfère la matrice ainsi obtenue sur un support final en polyéthylène. On découpe alors des formes de dimensions appropriées que l'on conditionne en sachets.

On obtient un système dont la quantité initiale de 17-p-oestradiol est de 463,6 µg/cm².

### Essais

Certains des résultats obtenus dans le cadre des essais comparatifs ont été regroupés dans les figures 1 à 6.

On a ainsi réalisé à partir des dispositifs décrits dans les exemples fournis ci-dessus des mesures ex-vivo de quantités d'hormone(s) libérées en 24 heures ou 48 heures sur divers modèles de peau et calculé les rendements correspondants. La comparaison avec d'autres systèmes déjà connus illustre de façon claire les avantages des dispositifs selon l'invention.

Pour cela on a effectué des tests de perméation ex-vivo sur peau abdominale de souris "nude" mâles selon le protocole suivant.

La mesure des quantités d'hormones libérées par un dispositif transdermique d'une surface de 2,54 cm² préalablement découpé à l'emporte pièce et déposé sur un disque de 3,14 cm² de peau abdominale de souris "nude" mâles est effectuée au moyen d'une cellule statique en verre, thermostatée à 37°C et ayant un compartiment récepteur d'un volume de 11,5 ml contenant comme phase réceptrice un mélange sérum physiologique/PEG₄₀₀ (75/25) V/V.

On effectue des prélèvements dans les solutions réceptrices à 2, 4, 6, 8, 12, 16, 20 et 24 heures que l'on dose par chromatographie liquide.

Compte-tenu de la variabilité des résultats liés à la perméabilité intrinsèque des échantillons de peau, chaque essai de perméation pour un échantillon de dispositif transdermique est réalisé sur un nombre minimum de 3 à 5 échantillons de peau.

On donne un résultat, qui est la moyenne pour chaque dispositif, obtenu à partir de ces essais.

Le rapport entre cette valeur moyenne des quantités d'hormone(s) libérées en fin de cinétique en 24 heures et la quantité initiale d'hormone(s) contenues dans le dispositif permet d'évaluer le rendement en 24 heures des systèmes transdermiques selon l'invention.

Les résultats obtenus sont rassemblés dans le tableau I.

On a aussi déterminé les quantités libérées en 24 heures par rapport à des dispositifs décrits dans l'art antérieur. On a ainsi réalisé suivant le même protocole que précédemment des tests de perméation cutanée ex-vivo sur peau abdominale de souris "nude" mâles de différents dispositifs selon l'invention contenant du 17-β-oestradiol et d'un système matriciel auto-adhésif (produit de comparaison CP4) du même type que ceux décrits dans la demande de brevet FR 2 612 785.

Les quantités libérées à 24 heures et les rendements calculés sont rassemblées dans le tableau II.

Enfin on a effectué des mesures comparatives vis-à-vis du seul système connu, actuellement commercialisé et qui contient à la fois un oestrogène et un progestatif, à savoir le produit ESTRAGEST^{©} de la société CIBA-GEIGY Ce produit est aussi le seul aujourd'hui disponible commercialement à contenir un progestatif bien que ce dernier soit en combinaison avec le 17-β-oestradiol. On applique le même protocole que précédemment pour effectuer des tests de perméation cutanée ex-vivo sur peau abdominale de souris "nude" mâles. On mesure à nouveau les quantités libérées en 24 heures et on calcule les rendements suivants le même principe.

Dans le cas du produit ESTRAGEST^{®} le dispositif est formé de deux réservoirs accolés contenant au total 10 mg de 17-β-oestradiol et 30 mg de NETA, chaque réservoir contenant un mélange de 5 mg de 17-p-oestradiol et 15 mg de NETA.

On effectue alors les mesures de perméation cutanée suivant le même protocole sur un seul des deux réservoirs placé sur un échantillon de peau de 3,14 cm².

Les quantités initiales de chaque hormone contenue dans ce réservoir sont ramenées à la quantité initiale de ladite hormones par unité de surface exprimée en µg/cm².

Dans un réservoir, on trouve en moyenne une valeur initiale d'hormone par unité de surface de (1590 + 30) µg/cm² de 17-p-oestradiol et de (4770 ± 30) µg/cm² de NETA.

De même, le rapport entre respectivement (i) la valeur moyenne des quantités de 17-p-oestradiol ou de NETA libérées en 24 heures et (ii) les quantités initiales contenues dans le réservoir permet d'obtenir les rendements en 24 heures en 17-p-oestradiol et NETA.

Dans un premier temps on a comparé des systèmes matriciels selon l'invention contenant du 17-β-oestradiol et du NETA au système transdermique connu ESTRAGEST^{®} précité. Les résultats obtenus ont été consignés dans le tableau III ci-après.

Dans une deuxième étape, on a aussi effectué des mesures comparatives ex-vivo sur un autre modèle de peau, la peau d'oreille de porc, qui donne en général des résultats ex-vivo plus proches de ceux obtenus par des mesures de tests de perméation ex-vivo sur peau humaine.

Le protocole opératoire de ces mesures est identique à celui des mesures ex-vivo sur peau abdominale de souris "nude". On a cette fois un disque de 3,14 cm² de peau d'oreille de porc. La phase réceptrice est toujours un mélange sérum physiologique/PEG₄₀₀ (75/25) VN. En revanche, on a effectué ici des prélèvements durant 48 heures.

Le résultat obtenu est toujours la moyenne obtenue pour chaque dispositif testé sur un nombre minimum de 3 à 5 échantillons de peau.

Le rapport entre (i) cette valeur moyenne et la quantité d'hormones libérées en fin de cinétique en 48 heures, et (ii) la quantité initiale d'hormone contenue dans le dispositif, permet d'évaluer le rendement en 48 heures des systèmes transdermiques.

Les résultats correspondants ont été consignés dans les tableaux IV et V ci-après.

Dans le cadre de l'étude relative au tableau I on a déterminé les quantités libérées en 24 heures entre 2 dispositifs matriciels dont la matrice comprend le même couple plastifiant/promoteur mais un copolymère de nature différente. C'est un SIS dans les exemples 1 à 3 et un EVA dans les exemples CP1 à CP3.

L'analyse du tableau I montre que dans le cas du NETA, en comparant le produit de l'exemple 1 avec CP1, on a des rendements 2,5 fois supérieurs dans le cas du SIS par rapport à l'EVA avec le même couple plastifiant/promoteur, à savoir le couple PROCETYL^{@} 10/SURFADONE^{®} LP 100. On a aussi une quantité de NETA libérée supérieure pour le produit de l'exemple 1.

On retrouve des résultats similaires dans le cas du 17-β-oestradiol en comparant le produit de l'exemple 2 avec CP2, où le couple plastifiant/promoteur est le couple alcool oléique/crotamiton Dans ce cas, on a également un rendement 1,7 fois supérieur en faveur du SIS.

De même si on analyse les résultats obtenus en comparant le produit de l'exemple 3 avec CP3, dans lesquels intervient le couple alcool oléique/SURFADONE^{®} LP 300, on obtient alors un rendement en 17-p-oestradiol 4 fois supérieur dans le cas du SIS (exemple 3) par rapport à l'EVA (CP3). Dans ce cas la quantité de 17-p-oestradiol libérée est 2,2 fois supérieure. On a donc avec le SIS un système matriciel transdermique qui permet une meilleure libération qu'avec l'EVA pourtant associé au même couple plastifiant/promoteur.

L'ensemble des résultats du tableau I montre donc que l'on a des rendements pour les dispositifs selon l'invention meilleurs grâce à l'association spécifique du matériau SIS et d'un couple plastifiant/promoteur selon l'invention, ainsi que des quantités libérées égales ou supérieures.

Dans le cadre de l'étude relative au tableau Il on a comparé des dispositifs selon l'invention par rapport à un dispositif identique à ceux décrits dans la demande de brevet FR-A-1 612 785.

L'analyse de ce tableau montre que dans tous les cas les quantités de 17-p-oestradiol libérées sont supérieures à celle du dispositif CP4 selon la demande de brevet publiée FR 2-612 785. Ce résultat est parfaitement illustré par la figure 1 qui compare les produits des exemples 4 et CP4.

Au niveau des rendements on constate que, par rapport au dispositif CP4, on a respectivement :
un rendement 7 fois supérieur pour l'exemple 2,
un rendement 13 fois supérieur pour l'exemple 3,
un rendement 7 fois supérieur l'exemple 6,
un rendement 10 fois supérieur pour l'exemple 7,
un rendement 6 fois supérieur pour l'exemple 4,

En conclusion dans tous les cas, quel que soit le couple plastifiant/promoteur utilisé, alcool oléique/crotamiton pour les produits des exemples 2 et 6, alcool oléique/SURFADONE^{®} LP 300 pour les produits des exemples 3 et 7, ou LABRAFIL^{®} 1944 Cs/crotamiton pour le produit de l'exemple 4, on a toujours des rendements beaucoup plus importants (de l'ordre de 6 à 13 fois) que ceux de CP4.

Les quantités libérées sont elles presque toujours environ 2,5 fois supérieures à celle de la matrice d'EVA correspondante.

Dans le cadre de l'étude relative au tableau III, on a comparé des systèmes matriciels selon l'invention contenant les deux hormones : le 17-β-oestradiol et le NETA au produit ESTRAGEST^{®.} Les résultats du tableau III montrent que dans tous les cas (i) les quantités de 17-p-oestradiol libérées en 24 heures sont environ entre 2 et 3 fois supérieures à celles du produit ESTRAGEST^{®} et (ii) les quantités de NETA libérées en 24 heures sont équivalentes ou supérieures à celles du produit comparatif ESTRAGEST^{®.}

De même les rendements aussi bien pour le 17-p-oestradiol que pour le NETA sont toujours très largement supérieurs à ceux du système ESTRAGEST^{®.} On constate plus précisément que par rapport à ce dernier on a respectivement, pour le 17-β-oestradiol :
un rendement 90 fois supérieur pour l'exemple 10,
un rendement 72 fois supérieur pour 1 ' exemple 11,
un rendement 47 fois supérieur pour l'exemple 12,

et on a respectivement, pour le NETA :
un rendement 25 fois supérieur pour l'exemple 10,
un rendement 20 fois supérieur pour l'exemple 11,
un rendement 19 fois supérieur pour l'exemple 12,

Ces différences très importantes mettent en évidence les avantages des systèmes matriciels transdermiques selon l'invention vis-à-vis du seul système transdermique combiné qui soit commercialisé à ce jour.

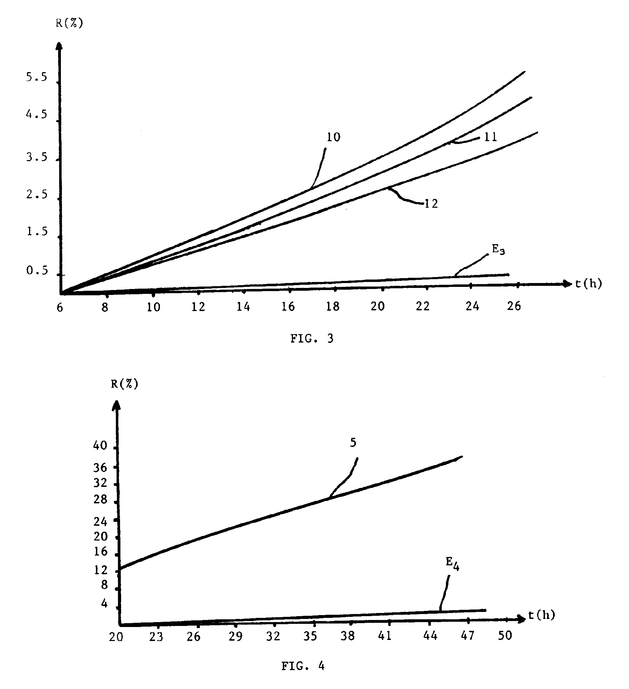

Ces différences sont corroborées par les figures 2 et 3 qui représentent le rendement en % de 17-β-oestradiot en fonction du temps (courbe 11) et le rendement en % de NETA en fonction de temps (courbe 12).

On constate aussi que dans ce cas quel que soit le promoteur associé au plastifiant (ici le PROCETYL^{®} 10) on a toujours avec le crotamiton ou une N-alkyl-2-pyrrolidone (la SURFADONE^{®} LP 300 ou la SURFADONE^{@} LP 100) des résultats remarquables.

Dans le cadre des études effectuées sur peau de porc (voir tableaux IV et V), on a comparé les dispositifs selon l'invention au produit ESTRAGEST^{@}.

Le tableau IV, relatif à la libération et au rendement du 17-β-oestradiol montre que l'on a avec ce modèle de peau une quantité de 17-β-oestradiol libérée en 48 heures et un rendement très supérieurs à ceux du dispositif ESTRA-GEST^{®}, en ce qui concerne le produit de l'exemple 5 selon l'invention. Ainsi la quantité libérée est 3 fois supérieure par rapport au produit ESTRAGEST^{®.}

La figure, 4 qui représente le rendement (%) de 17-β-oestradiol en fonction du temps pour le produit de l'exemple 5 et ledit ESTRAGEST^{®} illustre également ce résultat.

Le résultat du tableau IV met en évidence que, par rapport au dispositif ESTRAGEST^{@}, on a un rendement 57 fois supérieur pour l'exemple 5.

Ces valeurs toujours aussi importantes confirment donc les résultats déjà obtenus pour les tests de perméation cutanée ex-vivo sur peau abdominale de souris sur ce nouveau modèle de peau de porc.

Les résultats, qui ont été obtenus démontrent à nouveau les avantages du couple plastifiant/promoteur, où le plastifiant est l'oléate de péglicol (LABRAFIL 1944,Cs) et le promoteur la SURFADONE^{®} LP 300, avec le SIS.

Dans le tableau V, on a comparé sur peau d'oreille de porc, des dispositifs comportant les deux hormones, le 17-β-oestradiol et le NETA, selon l'invention (produits des exemples 8, 9 et 13) par rapport au produit ESTRAGEST^{®.}

Sur ce modèle de peau avec un système matriciel transdermique selon l'invention, on retrouve pour le 17-β-oestradiol des rendements très supérieurs à ceux du dispositif ESTRAGEST^{®.}

La figure 5 illustre aussi même phénomène en ce qui concerne les quantités de 17-p-oestradiol libérées en 48 heures.

Le tableau V montre en particulier que, par rapport au produit ESTRAGEST^{®}, on a respectivement un rendement 138 fois supérieur pour le produit de l'exemple 9, un rendement 72 fois supérieur pour le produit de l'exemple 8 et un rendement 107 fois supérieur pour le produit de l'exemple 13.

De même dans le cas du NETA on trouve aussi sur ce modèle de peau des rendements très supérieurs à ceux du dispositif ESTRAGEST^{®.}

Ceci est parfaitement illustré par la figure 6 qui représente le rendement (%) en fonction du temps, même si le phénomène est moins marqué qu'avec le 17-β-oestradiol, car on sait que le NETA passe plus difficilement la barrière cutanée.

Les résultats du tableau V montrent que l'on a, par rapport au dispositif ESTRAGEST^{®}, un rendement 21 fois supérieur pour le produit de l'exemple 8, un rendement 35 fois supérieur pour le produit de l'exemple 9 et un rendement 25 fois supérieur pour le produit de l'exemple 13.

Quand, dans le couple plastifiant/promoteur, le plastifiant est représenté par le LAUROGLYCOL^{®} et le promoteur par le crotamiton (exemple 9) ou une N-alkyl-2-pyrrolidone (SURFADONE^{©} LP 300 pour l'exemple 8 ou SURFA-DONE^{O} LP 100 pour l'exemple 13) leur association avec un matériau SIS permet donc à nouveau d'obtenir des résultats encore plus remarquables.

En conclusion l'ensemble des résultats donnés ci-dessus démontre les avantages indiscutables du système matriciel transdermique selon t'invention, sur différents modèles de peau par des tests ex-vivo, par rapport au seul produit commercialisé contenant un oestrogène et un progestatif, et à d'autres dispositifs matriciels auto-adhésifs décrits dans l'art antérieur.

Ils montrent en particulier que, en association avec un copolymère SIS les couples plastifiant/promoteur de l' invention permettent d'obtenir des rendements supérieurs qui sont dans certains cas exceptionnels.

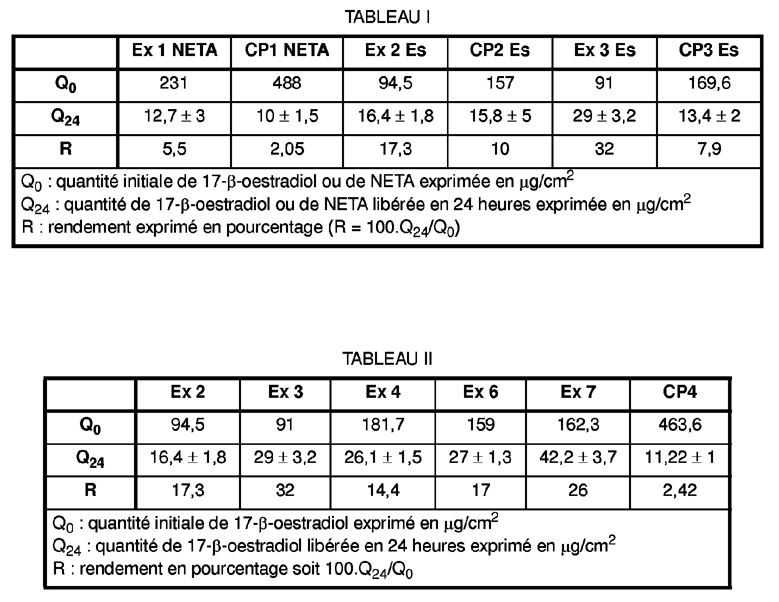

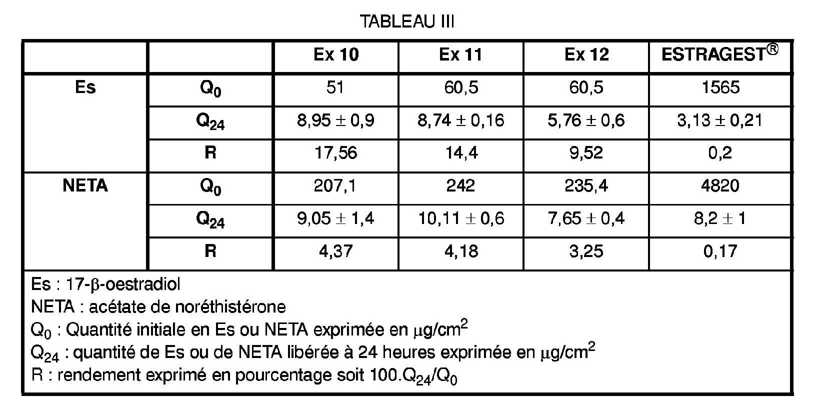

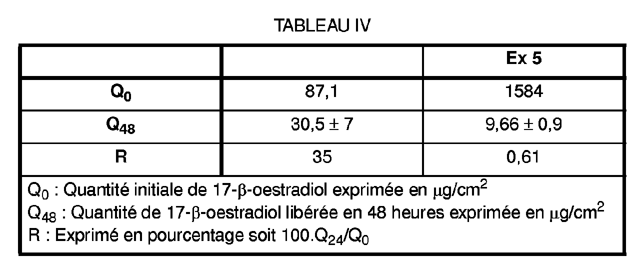

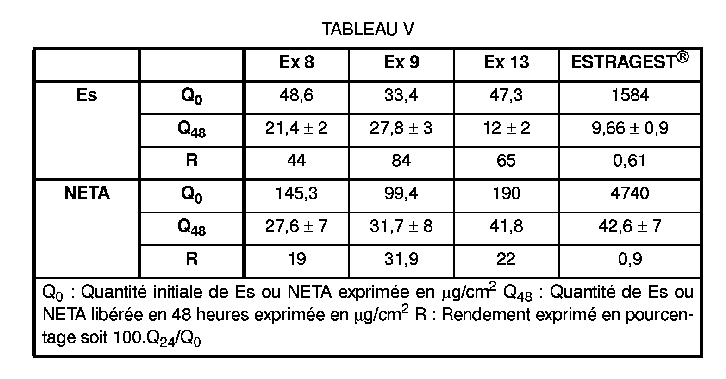

## Revendications

comprend :
(a) 20 à 42 parties en poids d'un copolymère tribloc poly(styrène-isoprène-styrène),
(b) 35 à 55 parties en poids d'une résine tackifiante,
(c) 5 à 25 parties en poids d'un agent plastifiant choisi parmi l'alcool oléique, le 5-oléate de péglicol, le laurate de propylèneglycol ou un éther polypropox^{y}lé de l'alcool cétylique,
(d) 5 à 15 parties en poids d'au moins un composé choisi parmi
- le crotamiton, et
- les 2-pyrrolidones N-substituées de formule I, 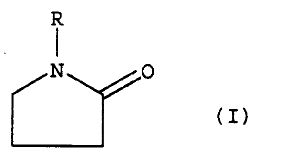 où R représente un groupe alkyle en C₁-C₁₅, le groupe cyclohexyle ou le groupe 2-hydroxyéthyle,
(e) 0,01 à 1 parties en poids d'un agent stabilisant, et,
(f) 0,1 à 5 parties en poids d'une hormone choisie parmi l'ensemble constitué par les composants oestrogènes, les composants progestatifs et leurs mélanges.

2. Système suivant la revendication 1 caractérisé en ce que l'agent plastifiant est l'alcool oléique.

3. Système suivant la revendication 1 caractérisé en ce que l'agent plastifiant est le 5.oléate de péglicol.

4. Système suivant la revendication 1 caractérisé en ce que l'agent plastifiant est le laurate de propylène glycol.

5. Système suivant la revendication 1 caractérisé en ce que l'agent plastifiant est un éther polypropoxylé de l'alcool cétylique, qui peut contenir notamment 10 moles d'oxyde de propylène pour 1 mole d'alcool cétylique.

6. Système suivant la revendication 1 caractérisé en ce que le composé promoteur de la perméation cutanée est choisi parmi l'ensemble constitué par le crotamiton et les composés 2-pyrrolidones N-substituées de formule I dans lequel le groupe R représente un groupe alkyle en C₁-C₁₅, le groupe cyclohexyle ou le groupe 2-hydroxyéthyle.

7. Système suivant la revendication 1 caractérisé en ce que le copolymère tribloc poly(styrène-isoprène-styrène) présente une teneur en styrène comprise entre 14 et 50 % en poids par rapport au poids dudit copolymère, et de préférence une teneur comprise entre 17 et 47 % en poids.

8. Système suivant la revendication 1 caractérisé en ce que l'oestrogène est le 17-p-oestradiol.

9. Système suivant la revendication 1 caractérisé en ce que le progestatif est l'acétate de noréthistérone.

10. Système suivant la revendication 1, caractérisé en ce que sa matrice contient pour un total de 100 parties en poids
(a) 37,3 parties en poids de copolymère tribloc poly(styrène-isoprène-styrène),
(b) 38 parties en poids de résine tackifiante,
(c) 15 parties en poids de laurate de propylène glycol,
(d) 7 parties en poids de N-octyl-2-pyrrolidone,
(e) 0,2 partie en poids d'agent stabilisant,
(f) 0,5 partie en poids de 17-¡3-oestradiol, et
(g) 2 parties en poids d'acétate de noréthistérone.

11. Procédé pour la préparation d'un système matriciel transdermique suivant l'une quelconque des revendications 1 à 10, ledit procédé étant caractérisé en ce qu'il comprend les étapes consistant à :
(a) mélanger le polymère SIS, l'agent stabilisant et la résine tackifiante à une température supérieure à 110°C,
puis homogénéiser le mélange résultant;
(β) incorporer dans le mélange homogène ainsi obtenu le ou les promoteurs et le plastifiant à une température comprise entre 80 et 110°C, puis homogénéiser le mélange résultant;
(y) incorporer dans le mélange homogène ainsi obtenu l'hormone choisie parmi l'ensemble constitué par les composants oestrogènes, les composants progestatifs et leurs mélanges, puis homogénéiser le mélange résultant;
(8) enduire le mélange homogène ainsi obtenu, à une température comprise entre 80 et 130°C, sur un support temporaire antiadhérent de façon à obtenir un revêtement sur ledit support de 50 à 300 g/m²; et,
(e) transférer ledit revêtement sur un support définitif.

12. Procédé pour la préparation d'un système matriciel transdermique suivant l'une quelconque des revendications 1 à 10, ledit procédé étant caractérisé en ce qu'il comprend les étapes consistant à :
(a) mélanger le polymère SIS, l'agent stabilisant puis la résine tackifiante dans un solvant dudit polymère SIS,
à une température inférieure à la température d'ébullition dudit solvant, puis homogénéiser le mélange résultant;
(β) incorporer dans le mélanne ainsi obtenu le ou les promoteurs et le plastifiant, puis homogénéiser le mélange résultant;
(y) incorporer dans le mélange homogène ainsi obtenu l'hormone choisie parmi l'ensemble constitué par les composants oestrogènes, les composants progestatifs et leurs mélanges, puis homogénéiser le mélange résultant;
(8) enduire le mélange homogène ainsi obtenu, à température ambiante, sur un support temporaire antiadhérent de façon à obtenir un revêtement sur ledit support de 50 à 300 glm², et,
(e) évaporer le solvant en chauffant ledit revêtement à une température supérieure au point d'ébullition dudit solvant, puis transférer ledit revêtement sur un support définitif.

## Claims

1. A self-adhesive transdermal matrix system for the administration of a hormone by percutaneous route, said matrix system, which has a support and a self-adhesive matrix, comprises a matrix containing :
(a) 20 to 42 parts by weight of a poly(styrene-isoprene-styrene) triblock copolymer,
(b) 35 to 55 parts by weight of a tackifying resin.
(c) 5 to 25 parts by weight of a plasticizing agent selected from the group consisting of oleic alcohol, peglicol 5-oleate, propyleneglycol laurate or a polypropoxyl ether of cetyl alcohol,
(d) 5 to 15 parts by weight of at least one compound selected from the group consisting of:
- crotamiton, and
- N-substituted 2-pyrrolidones of formula I, 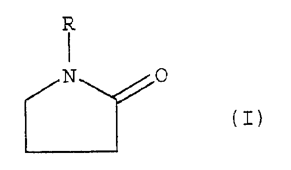 wherein R represents a (C₁-C₁₅)-alkyl, cyclohexyl or 2-hydroxyethyl group,
(e) 0,01 to 1 part by weight of a stabilizing agent and,
(f) 0,1 to 5 parts by weight of a hormone selected from the group consisting of oestrogen components, progestogen components and mixtures thereof.

2. A system as claimed in claim 1 wherein the plasticizing agent is oleic alcohol.

3. A system as claimed in claim 1 wherein the plasticizing agent is peglicol 5-oleate.

4. A system as claimed in claim 1 wherein the plasticizing agent is propylene glycol laurate.

5. A system as claimed in claim 1 wherein the plasticizing agent is a polypropoxyl ether of cetyl alcohol, which can in particular contain 10 moles of propylene oxide per 1 mole of cetyl alcohol.

6. A system as claimed in claim 1 wherein the compound which promotes cutaneous permeation is selected from the group consisting of crotamiton and N-substituted 2- pyrrolidone compounds of formula I in which the R group represents a (C₁-C₁₅)-alkyl, cyclohexyl or 2-hydroxyethyl group.

7. A system as claimed in claim 1 wherein the poly(styrene-isoprene-styrene) triblock copolymer has a styrene content of between 14 and 50 % by weight with respect to the weight of said copolymer, and preferably a content lying between 17 and 47% by weight.

8. A system as claimed in claim 1 wherein the oestrogen is 17-β-oestradiol.

9. A system as claimed in claim 1 wherein the progestogen is norethisterone acetate.

10. A system as claimed in claim 1 wherein its matrix contains per total 100 parts by weight:
(a) 37.3 parts by weight of the poly(styrene-isoprene-styrene) triblock copolymer,
(b) 38 parts by weight of tackifying resin,
(c) 15 parts by weight of propylene glycol laurate,
(d) 7 parts by weight of N-octyl-2-pyrrolidone,
(e) 0.2 part by weight of a stabilizing agent,
(f) 0.5 part by weight of 17-β-oestradiol, and
(g) 2 parts by weight of norethisterone acetate.

11. A method for preparing a transdermal matrix system as claimed in any one of claims 1 to 10, said method comprising the steps consisting of:
(a) mixing the SIS polymer, stabilizing agent and tackifying resin at a higher temperature than 110°C, then homogenizing the resulting mixture;
(β) incorporating into the homogeneous mixture thus obtained the promoter(s) and the plasticizer at a temperature of between 80 and 110°C then homogenizing the resulting mixture;
(y) incorporating into the homogeneous mixture thus obtained the hormone selected from the group consisting of oestrogen components, progestogen components and mixtures thereof, then homogenizing the resulting mixture;
(6) coating the homogeneous mixture thus obtained, at a temperature of from 80 to 130°C, onto a temporary anti-adherent support in such manner as to obtain a coating on said support of 50 to 300 g/m²; and,
(c) tranferring said coating to a final support.

12. A method for preparing a transdermal matrix system as claimed in any one of claims 1 to 10, said method comprising the steps consisting of:
(a) mixing the SIS polymer, stabilizing agent then the tackifying resin in a solvent of said SIS polymer, at a lower temperature than the boiling point temperature of said solvent, then homogenizing the resulting mixture;
(β) incorporating into the mixture thus obtained the promoter(s) and the plasticizer, then homogenizing the resulting mixture;
(y) incorporating into the homogeneous mixture thus obtained the hormone selected from the group consisting of oestrogen components, progestogen components and mixtures thereof, then homogenizing the resulting mixture;
(6) coating the homogeneous mixture thus obtained, at room temperature, onto a temporary anti-adherent support in such manner as to obtain a coating on said support of 50 to 300 g/m²; and,
(a) evaporating the solvent by heating said coating to a higher temperature than the boiling point temperature of said solvent, then transferring said coating to a final support.

## Patentansprüche

1. Transdermales selbstklebendes Matrixsystem zur perkutanen Verabreichung eines Hormons aus einem Träger und einer selbstklebenden Matrix, dadurch gekennzeichnet, daß die Matrix umfaßt:
(a) 20 bis 42 Gewichtsteile eines Styrolllsopren/Styrol-Triblockcopolymers,
(b) 35 bis 55 Gewichtsteile eines Klebeharzes,
(c) 5 bis 25 Gewichtsteile eines Weichmachers ausgewählt aus Oleinalkohol, Peglicol-5-oleat, Propylenglykollaurat oder einem polypropoxylierten Ether von Cetylalkohol,
(d) 5 bis 15 Gewichtsteile von mindestens einer Verbindung ausgewählt aus
- Crotamiton, und
- den N-substituierten 2-Pyrrolidonen der Formel I, 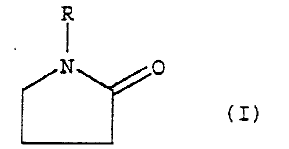 worin R eine C₁-C₁₅-Alkylgruppe, die Cyclohexylgruppe oder die 2-Hydroxyethylgruppe bedeutet,
(e) 0,01 bis 1 Gewichtsteile eines Stabilisators, und
(f) 0,1 bis 5 Gewichtsteile eines Hormons ausgewählt aus der Gruppe bestehend aus Östrogenen, Gestagenen und deren Mischungen.

2. System nach Anspruch 1, dadurch gekennzeichnet, daß der Weichmacher Oleinalkohol ist.

3. System nach Anspruch 1, dadurch gekennzeichnet, daß der Weichmacher Peglicol-5-oleat ist.

4. System nach Anspruch 1, dadurch gekennzeichnet, daß der Weichmacher Propylenglykollaurat ist.

5. System nach Anspruch 1, dadurch gekennzeichnet, daß der Weichmacher ein polypropoxylierter Ether von Cetylalkohol ist, der insbesondere 10 mol Propylenoxid pro 1 mol Cetylalkohol enthält.

6. System nach Anspruch 1, dadurch gekennzeichnet, daß der Hautpermeationspromotor ausgewählt ist aus der Gruppe bestehend aus Crotamiton und den N-substituierten 2-Pyrrolidonen der Formel I, worin R eine C₁-C₁₅-Alkylgruppe, die Cyclohexylgruppe oder die 2-Hydroxyethylgruppe bedeutet.

7. System nach Anspruch 1, dadurch gekennzeichnet, daß das Styrol/isopren/Styrol-Tribiockcopolymer einen Styrolgehalt zwischen 14 und 50 Gew. -%, bezogen auf das Gewicht des Copolymers, aufweist, und vorzugsweise einen Gehalt zwischen 17 und 47 Gew.-%.

8. System nach Anspruch 1, dadurch gekennzeichnet, daß das Östrogen 17-[3-Östradiol ist.

9. System nach Anspruch 1, dadurch gekennzeichnet, daß das Gestagen Norethisteronacetat ist.

10. System nach Anspruch 1, dadurch gekennzeichnet, daß die Matrix in insgesamt 100 Gewichtsteilen enthält:
(a) 37,3 Gewichtsteile Styrolllsopren/Styrol-Triblockcopolymer,
(b) 38 Gewichtsteile Klebeharz,
(c) 15 Gewichtsteile Propylenglykollaurat,
(d) 7 Gewichtsteile N-Octyl-2-pyrrolidon,
(e) 0,2 Gewichtsteile Stabilisator,
(f) 0,5 Gewichtsteile 17-ß-Östradiol, und
(g) 2 Gewichtsteile Norethisteronacetat.

11. Verfahren zur Herstellung eines transdermalen Matrixsystems nach einem der Ansprüche 1 bis 10, dadurch gekennzeichnet, daß es die Stufen umfaßt:
(a) Mischen des SIS-Polymers, des Stabilisators und des Klebeharzes bei einer Temperatur oberhalb von 110 °C, und Homogenisieren der erhaltenen Mischung;
(ß) Zusetzen zur so erhaltenen homogenisierten Mischung den oder die Promotoren und den Weichmacher bei einer Temperatur zwischen 80 und 110 °C, und Homogenisieren der erhaltenen Mischung;
(y) Zusetzen zur so erhaltenen homogenisierten Mischung das Hormon ausgewählt aus der Gruppe bestehend aus Östrogenen, Gestagenen und deren Mischungen, und Homogenisieren der erhaltenen Mischung;
(δ) Aufbringen der so erhaltenen homogenisierten Mischung bei einer Temperatur zwischen 80 und 130 °C auf einen provisorischen nichtklebenden Träger auf eine Weise, daß man auf dem Träger einen Überzug von 50 bis 300 g/m² erhält; und,
(c) Übertragen des Überzugs auf einen endgültigen Träger.

12. Verfahren zur Herstellung eines transdermalen Matrixsystems nach einem der Ansprüche 1 bis 10, dadurch gekennzeichnet, daß es die Stufen umfaßt:
(a) Mischen des SIS-Polymers, des Stabilisators und des Klebeharzes in einem Lösungsmittel für das SIS-Polymer bei einer Temperatur unterhalb der Temperatur des Siedepunktes des Lösungsmittels, und Homogenisieren der erhaltenen Mischung;
(β) Zusetzen zur so erhaltenen Mischung den oder die Promotoren und den Weichmacher, und Homogenisieren der erhaltenen Mischung;
(y) Zusetzen zur so erhaltenen homogenisierten Mischung das Hormon ausgewählt aus der Gruppe bestehend aus Östrogenen, Gestagenen und deren Mischungen, und Homogenisieren der erhaltenen Mischung;
(8) Aufbringen der so erhaltenen homogenisierten Mischung bei Raumtemperatur auf einen provisorischen nicht klebenden Träger auf eine Weise, daß man auf dem Träger einen Überzug von 50 bis 300 g/m² erhält; und
(c) Abdampfen des Lösungsmittels durch Erhitzen des Überzuges auf eine Temperatur oberhalb des Siedepunktes des Lösungsmittels, und Übertragen des Überzugs auf einen endgültigen Träger.
